Europäisches Patentamt

European Patent Office

Office européen des brevets

(19)

(11) Veröffentlichungsnummer: **0 161 504 B1**

(12) # EUROPÄISCHE PATENTSCHRIFT

(45) Veröffentlichungstag der Patentschrift: **06.11.91**

(21) Anmeldenummer: **85104501.3**

(22) Anmeldetag: **13.04.85**

Die Akte enthält technische Angaben, die nach dem Eingang der Anmeldung eingereicht wurden und die nicht in dieser Patentschrift enthalten sind.

(51) Int. Cl.⁵: **C07K 7/10, C07K 15/26, C12N 15/00, C12P 19/34, C12N 1/20, A61K 37/66, //C12R1/19**

(54) **Herstellung von Polypeptiden mit Human-Gammainterferon-Aktivität.**

(30) Priorität: **19.04.84 DE 3414831**

(43) Veröffentlichungstag der Anmeldung:
**21.11.85 Patentblatt 85/47**

(45) Bekanntmachung des Hinweises auf die Patenterteilung:
**06.11.91 Patentblatt 91/45**

(84) Benannte Vertragsstaaten:
**AT BE CH DE FR GB IT LI LU NL SE**

(56) Entgegenhaltungen:
**EP-A- 89 676**
**EP-A- 95 350**
**EP-A- 103 898**
**EP-A- 146 354**

**WO 83/04053**

**The Biology of Interferon System, 1983, E.DeMaeyer & A.Schellekens Editors; K.Alton et al., pp. 119-128**

**JOURNAL OF BIOLOGICAL CHEMISTRY, vol.**

**259, no. 11; E.RINDERKNECHT et al., pp. 6790-6797**

(73) Patentinhaber: **HOECHST AKTIENGESELL-SCHAFT**
**Postfach 80 03 20**
**W-6230 Frankfurt am Main 80(DE)**

(72) Erfinder: **Engels, Joachim, Dr.**
**Feldbergstr. 1**
**W-6242 Kronberg/Taununs(DE)**
Erfinder: **Leineweber, Michael, Dr.**
**Heimchenweg 74**
**W-6230 Frankfurt am Main 80(DE)**
Erfinder: **Uhlmann, Eugen, Dr.**
**Washington Street 287**
**Belmont, MA 02178(US)**
Erfinder: **Ulmer, Wolfgang, Dr.**
**Dr. Fuchs-Str. 8**
**W-6382 Friedrichsdorf(DE)**

## Beschreibung

Die Erfindung ist in den Patentansprüchen definiert. Sie betrifft insbesondere ein Verfahren zur Herstellung von Polypeptiden, welche die biologische und immunologische Aktivität von Human-Gammainterferon zeigen, chemisch synthetisierte Gene, die diese Peptide codieren sowie geeignete Vektorkonstruktionen und Wirtsorganismen zur Expression dieser Polypeptide. Die Erfindung bezieht sich weiterhin auf neue Polypeptide, aus denen im Unterschied zu Gammainterferon Partialsequenzen deletiert sind, welche für die biologische Aktivität nicht essentiell sind. Diese neuen Polypeptide besitzen gegenüber Gammainterferon eine erhöhte Stabilität oder Löslichkeit und variieren in der Spezifität ihrer antiviralen Aktivität, sind jedoch - wie Gammainterferon - alle als antivirale, antitumorale, antineoplastische oder immunomodulierende Präparate einsetzbar.

Das Gammainterferon (früher Immuninterferon oder Typ II-Interferon; hier kurz als IFN-γ bezeichnet) wurde im Jahre 1965 von F. Wheelock entdeckt (Wheelock; Science 149 (1965), 310), welcher zeigte, daß IFN-γ bestimmte Zellen vor einer Virusinfektion schützen kann. Menschliches IFN-γ (Basiswissen s. W.E. Stewart, II, The Interferon System, Springer Verlag (2nd ed., 1981)) ist ein Polypeptid aus 146 Aminosäuren (Gray et al., Nature 295 (1982), 503), das von Natur aus glykosyliert ist. Das Glykoprotein besitzt ein Molekulargewicht von etwa 63 000 - 73 000 (Pestka et al., J. Biol. Chem. 258 (1983), 9706) und liegt in seiner funktionellen Form wahrscheinlich als Tetramer vor. Die Glykosylierung des IFN-γ ist für seine Funktionalität nicht erforderlich; so reduziert die Glykosidase-Behandlung von IFN-γ nicht seine antivirale Aktivität in humanen Fibroblasten-Zellkulturen (Kelker et al., J. Biol. Chem. 258 (1983), 8010).

Ferner ist IFN-γ im Gegensatz zu den Alphainterferonen und Betainterferon bei pH 2 instabil und wird auch durch Hitze (60° C) desaktiviert.

Die Gewinnung von Human-IFN-γ aus Zellkulturen menschlicher Zellinien oder aus Leukozyten (Blutkonserven) ist nur in schlechter Ausbeute und niedriger Produktreinheit möglich. Die Erfindung betrifft die gentechnologische Herstellung von Polypeptiden mit gammainterferonähnlichen Eigenschaften. Human-IFN-γ besitzt folgende Peptidsequenz (Devos et al., Nucl. Acids Research 10 (1982), 2487):

$$\text{Cys}^1 - \text{Tyr} - \text{Cys} - \text{Gln} - \text{Asp} - \text{Pro} - \text{Tyr} - \text{Val} - \text{Lys} - \text{Glu}^{10}-$$
$$\text{Ala} - \text{Glu} - \text{Asn} - \text{Leu} - \text{Lys} - \text{Lys} - \text{Tyr} - \text{Phe} - \text{Asn} - \text{Ala}^{20}-$$
$$\text{Gly} - \text{His} - \text{Ser} - \text{Asp} - \text{Val} - \text{Ala} - \text{Asp} - \text{Asn} - \text{Gly} - \text{Thr}^{30}-$$
$$\text{Leu} - \text{Phe} - \text{Leu} - \text{Gly} - \text{Ile} - \text{Leu} - \text{Lys} - \text{Asn} - \text{Trp} - \text{Lys}^{40}-$$
$$\text{Glu} - \text{Glu} - \text{Ser} - \text{Asp} - \text{Arg} - \text{Lys} - \text{Ile} - \text{Met} - \text{Gln} - \text{Ser}^{50}-$$
$$\text{Gln} - \text{Ile} - \text{Val} - \text{Ser} - \text{Phe} - \text{Tyr} - \text{Phe} - \text{Lys} - \text{Leu} - \text{Phe}^{60}-$$
$$\text{Lys} - \text{Asn} - \text{Phe} - \text{Lys} - \text{Asp} - \text{Asp} - \text{Gln} - \text{Ser} - \text{Ile} - \text{Gln}^{70}-$$
$$\text{Lys} - \text{Ser} - \text{Val} - \text{Glu} - \text{Thr} - \text{Ile} - \text{Lys} - \text{Glu} - \text{Asp} - \text{Met}^{80}-$$
$$\text{Asn} - \text{Val} - \text{Lys} - \text{Phe} - \text{Phe} - \text{Asn} - \text{Ser} - \text{Asn} - \text{Lys} - \text{Lys}^{90}-$$
$$\text{Lys} - \text{Arg} - \text{Asp} - \text{Asp} - \text{Phe} - \text{Glu} - \text{Lys} - \text{Leu} - \text{Thr} - \text{Asn}^{100}-$$
$$\text{Tyr} - \text{Ser} - \text{Val} - \text{Thr} - \text{Asp} - \text{Leu} - \text{Asn} - \text{Val} - \text{Gln} - \text{Arg}^{110}-$$
$$\text{Lys} - \text{Ala} - \text{Ile} - \text{His} - \text{Glu} - \text{Leu} - \text{Ile} - \text{Gln} - \text{Val} - \text{Met}^{120}-$$
$$\text{Ala} - \text{Glu} - \text{Leu} - \text{Ser} - \text{Pro} - \text{Ala} - \text{Ala} - \text{Lys} - \text{Thr} - \text{Gly}^{130}-$$
$$\text{Lys} - \text{Arg} - \text{Lys} - \text{Arg} - \text{Ser} - \text{Gln} - \text{Met} - \text{Leu} - \text{Phe} - \text{Arg}^{140}-$$
$$\text{Gly} - \text{Arg} - \text{Arg} - \text{Ala} - \text{Ser} - \text{Gln}^{146}.$$

Aus WO 83/04053 ist ein Derivat des Human-IFN-γ bekannt, bei dem die ersten drei Aminosäuren der vorstehend genannten Sequenz deletiert sind und wobei in Position 81 Lys anstelle von Asn in der natürlichen Sequenz steht. Diese Verbindung soll gegenüber dem unveränderten Molekül nur 60% Aktivität aufweisen.

Ein Aspekt der vorliegenden Erfindung betrifft die Herstellung von biologisch aktiven Human-IFN-γ-Teilsequenzen, insbesondere der Teilsequenzen 1-127, 5-146 und 5-127 der vorangehenden Sequenz.

Der genetische Code ist bekanntlich "entartet", d.h. daß nur für zwei Aminosäuren eine einzige Nucleotid-Sequenz codiert, während den restlichen 18 genetisch codierbaren Aminosäuren 2 bis 6 Tripletts zuzuordnen sind. Von den hierdurch gegebenen Variationsmöglichkeiten machen außerdem die Wirtszellen unterschiedlicher Spezies nicht immer den gleichen Gebrauch. Für die Synthese der Gene besteht somit eine unübersehbare Vielfalt von Codon-Möglichkeiten. Es wurde nun gefunden, daß die DNA-Sequenz I (Anhang), die für die gesamte Aminosäurensequenz 1-146 codiert, sowie die sich daraus ableitenden DNA-Sequenzen IA, IB und IC besonders vorteilhaft für die gentechnologische Synthese von Polypeptiden mit IFN-$\gamma$-Aktivität sind. An das 5'-Ende des codierenden Stranges der DNA-Sequenz I schließt sich das Codon für Methionin ("Triplett Nr. O") und davor eine "überhängende" DNA-Sequenz entsprechend einer Restriktionsendonuclease, beispielsweise Eco RI,an. Am 3'-Ende des codierenden Stranges dagegen befindet sich im Anschluß an ein Stop-Codon oder vorzugsweise zwei Stop-Codons - unmittelbar oder über eine dazwischen liegende DNA-Sequenz - die einem Restriktionsenzym entsprechende Sequenz, beispielsweise Sal I. Unterschiedliche Erkennungssequenzen gewährleisten die Insertion der DNA in Plasmide in der gewünschten Orientierung.

Am 5'-Ende des codierenden Stranges kann alternativ zu dem Codon für die Aminosäure Methionin eine Praesequenz (auch Signal- oder leader-Sequenz genannt) eines bakteriellen oder sonstigen wirtseigenen Proteins stehen (Übersichtsartikel: Perlman und Halvorson; J. Mol. Biol. 167 (1983), 391), welche die Sekretion des gewünschten Polypeptids aus dem Cytoplasma bewirkt und bei diesem Exkretionsprozeß von einer in der Wirtszelle natürlich vorkommenden Signal-Peptidase abgespalten wird.

Zwei interne singuläre Schnittstellen für die Restriktionsenzyme Bam HI bzw. Hind III (im Codon 34 bzw. 97 des codierenden Stranges oder im Codon 35 bzw. 98 des nichtcodierenden Strangs) ermöglichen die Subklonierung dreier Genfragmente IFN-I, IFN-II und IFN-III (siehe DNA-Sequenz II), die in gut untersuchte Klonierungsvektoren, wie etwa pBR 322 oder pUC 8, eingebaut werden können. Zusätzlich wurden innerhalb des Strukturgens eine Reihe von weiteren singulären Erkennungssequenzen für Restriktionsenzyme eingebaut, die einerseits einen Zugang zu Teilsequenzen des IFN-$\gamma$ schaffen und andererseits die Durchführung von Variationen erlauben:

| Restriktionsenzym | Schnitt nach Nucleotid-Nr. (codierender Strang) |
|---|---|
| Ava II[a] | 20 |
| Alu I[b] | 39 |
| Hinf I[a] | 134 |
| Dde I[c] | 159 |
| Aha III[c] | 199 |
| Taq I[c] | 294 |
| Aha III[d] | 327 |
| Sst I[a] | 357 |
| Bst NI[d] | 362 |
| Pst I[a] | 388 |
| Bbv I[a] | 398 |
| Sst II[a] | 430 |
| Dde I[d] | 444 |

a) singulär bezüglich der Gesamt-DNA-Sequenz I
b) singulär bezüglich der Teilsequenz IFN-I
c) singular bezüglich der Teilsequenz IFN-II
d) singulär bezüglich der Teilsequenz IFN-III

Die DNA-Sequenz I und die sich an den Enden anschließenden Sequenzen lassen sich aus 34 Oligonucleotiden mit einer Länge von 18 bis 33 Nucleotiden aufbauen (siehe DNA-Sequenz II),indem diese zunächst chemisch synthetisiert und dann über "sticky ends" von 4 bis 6 Nucleotiden enzymatisch verknüpft werden.

Bei der DNA-Sequenz I wurde weiterhin berücksichtigt, daß bei denjenigen Aminosäuren, denen mehrere Codons zuzuordnen sind, diese nicht gleichwertig sind, sondern vielmehr in der jeweiligen Wirtszelle wie E.coli unterschiedliche Präferenzen zeigen. Weiterhin wurden palindromische Sequenzen auf ein Mindestmaß reduziert.

Die Genstruktur der DNA-Sequenz I ist somit leicht aus relativ kleinen Bausteinen zugänglich, ermöglicht die Subklonierung dreier Genfragmente in gut bekannte Vektoren und erlaubt deren Kombination zum Gesamtgen sowie Veränderungen derselben. So können nach der Klonierung des Gens mit der DNA-Sequenz I aus dieser durch Spaltung mit bestimmten Restriktionsenzymen DNA-Teilsequenzen erhalten werden, insbesondere die Partialsequenzen IA, IB und IC, die für die Interferon-Teilsequenzen entsprechend der Aminosäuren 1-127, 5-146 und 5-127 codieren.

Als Beispiel für eine Teilsequenz steht die DNA-Sequenz IA, die zu einem Polypeptid mit den ersten 127 Aminosäuren des IFN-$\gamma$ führt, wobei die DNA-Sequenz I derart abgewandelt ist, daß an das Triplett Nr. 127 unmittelbar ein Stop-Triplett oder vorzugsweise zwei Stop-Tripletts sowie das überhängende Ende für ein Restriktionsenzym, beispielsweise Sal I, anschließen.

Durch Schneiden der DNA-Sequenz I mit der Restriktionsendonuclease Ava II und Ligation des dabei erhaltenen großen Fragments mit einer Adaptorsequenz erhält man dagegen eine DNA-Sequenz IB, bei der die Codons für die ersten vier Aminosäuren des IFN-$\gamma$ deletiert sind, d.h. vor Aminosäure Nr. 5 (Asparaginsäure) steht direkt Methionin. Aus dieser DNA-Sequenz IB läßt sich über die Pst I-Schnittstelle eine DNA-Sequenz IC generieren, die für ein Polypeptid mit den Aminosäuren 5-127 des IFN-$\gamma$ codiert.

Der Einbau der synthetischen Gene bzw. Genfragmente in Klonierungsvektoren, beispielsweise in die handelsüblichen Plasmide pUC 8 und pBR 322 bzw. andere allgemein zugängliche Plasmide wie ptac 11 und pKK 177.3, erfolgt in an sich bekannter Weise. Auch können die chemisch synthetisierten Gene zuvor mit geeigneten chemisch synthetisierten Kontrollregionen versehen werden, die eine Expression der Proteine ermöglichen. Hierzu kann auf das Lehrbuch von Maniatis (Molecular Cloning, Maniatis et al., Cold Spring Harbor, 1982) verwiesen werden. Die Transformation der so erhaltenen Hybridplasmide in geeignete Wirtsorganismen, vorteilhaft E. coli, ist ebenfalls an sich bekannt und in dem vorstehend genannten Lehrbuch eingehend beschrieben. Die Gewinnung des exprimierten Proteins und dessen Reinigung sind ebenfalls beschrieben (J.A. Georgiades, Texas Reports in Biology and Medicine 41 (1981) 179; Came and Carter (Editors), "Interferons and Their Applications", Springer-Verlag 1984).

Die erfindungsgemäß erhaltenen Polypeptide mit Gammainterferon-Aktivität gemäß den DNA-Sequenzen IA, IB und IC sind neu und Gegenstand der Erfindung. Dasselbe gilt für aus der neuen DNA-Sequenz I abgewandelte DNA-Sequenzen, daraus zugängliche Gammainterferon-Analoga, die Genfragmente IFN-I, IFN-II und IFN-III sowie deren Abwandlungen, die damit erhaltenen Hybridplasmide und die transformierten Wirtsorganismen.

Weitere Ausgestaltungen der Erfindung sind in den Patentansprüchen niedergelegt.

In den folgenden Beispielen werden noch einige Ausgestaltungen der Erfindung im einzelnen erläutert, woraus sich die Vielzahl der möglichen Abwandlungen und Kombinationen für den Fachmann ergeben. Prozentangaben beziehen sich hierbei auf das Gewicht, wenn nichts anderes angegeben ist.

Beispiele

1. Chemische Synthese eines einzelsträngigen Oligonucleotids

Am Beispiel des Genbausteins Ia, der die Nucleotide 1-23 des codierenden Strangs umfaßt, wird die Synthese der Genbausteine erläutert. Nach bekannten Methoden (M.J. Gait et al., Nucleic Acids Res. 8 (1980) 1081-1096)) wird das am 3'-Ende stehende Nucleosid, im vorliegenden Falle also Cytidin (Nucleotid Nr. 23), an Kieselgel ((R)FRACTOSIL, Firma Merck) über die 3'-Hydroxyfunktion covalent gebunden. Hierzu wird zunächst das Kieselgel unter Abspaltung von Ethanol mit 3-(Triethoxysilyl)-propylamin umgesetzt, wobei eine Si-O-Si-Bindung entsteht. Das Cytidin wird als N$^4$-Benzoyl-3'-O-succinoyl-5'-dimethoxytritylether in Gegenwart von Paranitrophenol und N,N'-Dicyclohexylcarbodiimid mit dem modifizierten Träger umgesetzt, wobei die freie Carboxygruppe der Succinoylgruppe den Aminorest der Propylaminogruppe acyliert.

In den folgenden Syntheseschritten wird die Basenkomponente als 5'-O-Dimethoxytrityl-nucleosid-3'-phosphorigsäuremonomethylester-dialkylamid oder -chlorid eingesetzt, wobei das Adenin als N$^6$-Benzoyl-Verbindung, das Cytosin als N$^4$-Benzoyl-Verbindung, das Guanin als N$^2$-Isobutyryl-Verbindung und das keine Aminogruppe enthaltende Thymin ohne Schutzgruppe vorliegen. 50 mg des polymeren Trägers, der 2 $\mu$mol Cytosin gebunden enthält, werden nacheinander mit den folgenden Agentien behandelt:

a) Nitromethan,
b) gesättigte Zinkbromidlösung in Nitromethan mit 1 % Wasser,
c) Methanol,
d) Tetrahydrofuran,
e) Acetonitril,
f) 40 $\mu$mol des entsprechenden Nucleosidphosphits und 200 $\mu$mol Tetrazol in 0,5 ml wasserfreiem Acetonitril (5 Minuten),

g) 20 % Acetanhydrid in Tetrahydrofuran mit 40 % Lutidin und 10 % Dimethylaminopyridin (2 Minuten),

h) Tetrahydrofuran,

i) Tetrahydrofuran mit 20 % Wasser und 40 % Lutidin,

j) 3 % Jod in Kollidin/Wasser/Tetrahydrofuran im Volumenverhältnis 5:4:1,

k) Tetrahydrofuran und

l) Methanol.

Unter "Phosphit" wird hierbei der Desoxyribose-3'-monophosphorigsäure-monomethylester verstanden, wobei die dritte Valenz durch Chlor oder eine tertiäre Aminogruppe, beispielsweise einen Morpholinorest, abgesättigt ist. Die Ausbeuten der einzelnen Syntheseschritte können jeweils nach der Detritylierungsreaktion b) spektrophotometrisch durch Messung der Absorption des Dimethoxytritylkations bei einer Wellenlänge von 496 nm bestimmt werden.

Nach abgeschlossener Synthese des Oligonucleotids werden die Methylphosphatschutzgruppen des Oligomers mit Hilfe von p-Thiokresol und Triethylamin abgespalten.

Anschließend wird durch 3-stündige Behandlung mit Ammoniak das Oligonucleotid vom festen Träger abgetrennt. Eine 2- bis 3-tägige Behandlung der Oligomeren mit konzentriertem Ammoniak spaltet die Aminoschutzgruppen der Basen quantitativ ab. Das so erhaltene Rohprodukt wird durch Hochdruckflüssigkeitschromatographie (HPLC) oder durch Polyacrylamid-Gelelektrophorese gereinigt.

Ganz entsprechend werden auch die übrigen Genbausteine Ib-IIII synthetisiert, deren Nucleotidfolge aus der DNA-Sequenz II hervorgeht.

2. Enzymatische Verknüpfung der einzelsträngigen Oligonucleotide zu den Genfragmenten IFN-I, IFN-II und IFN-III

Zur Phosphorylierung der Oligonucleotide am 5'-Terminus wurde je 1 nmol der Oligonucleotide Ia und Ib mit 5 nmol Adenosintriphosphat mit vier Einheiten T4-Polynucleotid-Kinase in 20 $\mu$l 50 mM Tris-HCl-Puffer (pH 7,6), 10 mM Magnesiumchlorid und 10 mM Dithiothreitol (DTT) 30 Minuten bei 37° C behandelt (C.C. Richardson, Progress in Nucl. Acids Res. 2 (1972) 825). Das Enzym wird durch fünfminütiges Erhitzen auf 95° C desaktiviert. Anschließend werden die Oligonucleotide Ia und Ib gegeneinander hybridisiert, indem man sie in wäßriger Lösung 2 Minuten auf 95° C erhitzt und dann langsam auf 5° C abkühlt.

Analog werden die Oligonucleotide Ic und Id, Ie und If, Ig und Ih sowie Ii und Ij phosphoryliert und paarweise hybridisiert. Für das Subfragment IFN-II werden die Oligonucleotide IIa mit IIb usw. bis IIk mit III und für das Subfragment IFN-III die Oligomeren IIIa mit IIIb usw. bis IIIk mit IIII phosphoryliert und paarweise hybridisiert.

Die so erhaltenen fünf Oligonucleotidpaare für das Genfragment IFN-I bzw. die sechs Oligonucleotidpaare für die Genfragmente IFN-II und IFN-III werden jeweils wie folgt ligiert:

Die doppelsträngigen Nucleotide werden vereinigt und in jeweils 40 $\mu$l 50 mM Tris-HCl-Puffer, 20 mM Magnesiumchlorid und 10 mM DTT mit Hilfe von 100 Einheiten T4-DNA-Ligase bei 15° C im Laufe von 16 Stunden ligiert.

Die Reinigung der Genfragmente IFN-I bis IFN-III erfolgt durch Gelelektrophorese auf einem 10%igen Polyacrylamidgel (ohne Harnstoffzusatz, 20 · 40 cm, 1 mm Dicke), wobei als Markersubstanz ØX 174 DNA (Fa. BRL), geschnitten mit Hinf I, oder pBR 322, geschnitten mit Hae III, diente.

3. Herstellung von Hybridplasmiden, die die Genfragmente IFN-I, IFN-II und IFN-III enthalten

a) Einbau des Genfragmentes IFN-I in pBR 322

Das handelsübliche Plasmid pBR 322 wird in bekannter Weise mit den Restriktionsendonucleasen Eco RI und Bam HI nach den Angaben der Hersteller geöffnet. Der Verdauungsansatz wird auf einem 5%igen Polyacrylamidgel durch Elektrophorese in bekannter Weise aufgetrennt und die Bruchstücke durch Anfärben mit Ethidiumbromid oder durch radioaktive Markierung ("Nick-Translation" nach Maniatis, a.a.O.) sichtbar gemacht. Die Plasmidbande wird anschließend aus dem Acrylamidgel ausgeschnitten und elektrophoretisch vom Polyacrylamid abgetrennt. Die Auftrennung des Verdauungsansatzes kann auch auf 2%igen niedrigschmelzenden Agarosegelen (wie in Beispiel 6a) beschrieben) erfolgen.

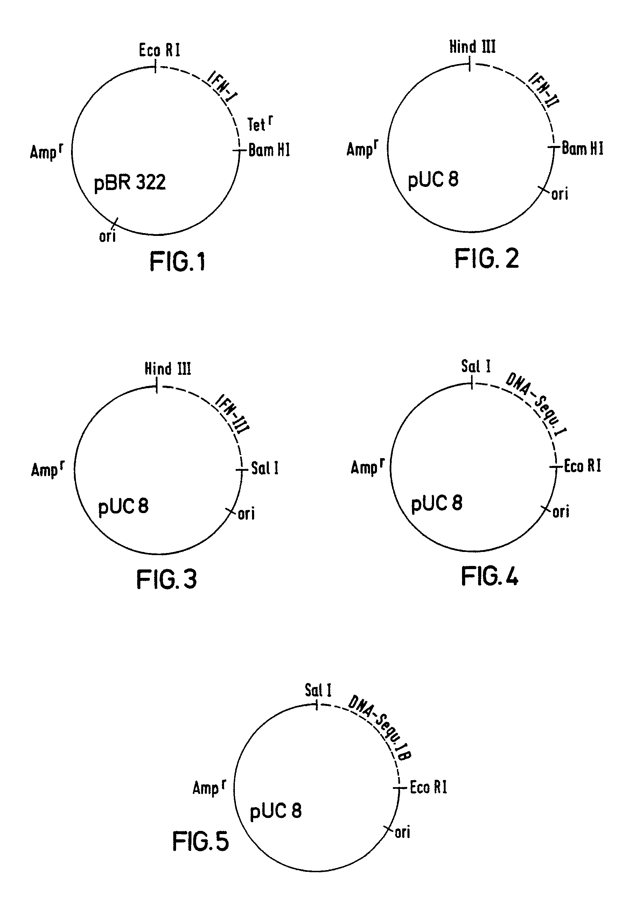

1 $\mu$g Plasmid wird dann mit 10 ng Genfragment IFN-I über Nacht bei 16° C ligiert. Man erhält das Hybridplasmid gemäß Figur 1.

b) Einbau des Genfragments IFN-II in pUC 8

Analog zu a) wird das handelsübliche Plasmid pUC 8 mit Bam HI und Hind III aufgeschnitten und mit dem Genfragment IFN-II ligiert. Man erhält das Hybridplasmid gemäß Figur 2.

c) Einbau des Genfragments IFN-III in pUC 8

Analog zu a) wird das Plasmid pUC 8 mit Hind III und Sal I aufgeschnitten und mit dem Genfragment IFN-III ligiert. Man erhält das Hybridplasmid gemäß Figur 3.

4. Synthese des kompletten Gens

a) Transformation und Amplifikation
Die so erhaltenen Hybridplasmide werden in E. coli transformiert. Hierzu wird der Stamm E. coli K 12 durch Behandlung mit einer 70 mM Calciumchloridlösung kompetent gemacht und mit der Suspension des Hybridplasmids in 10 mM Tris-HCl-Puffer (pH 7,5), der 70 mM an Calciumchlorid ist, versetzt. Die transformierten Stämme werden wie üblich unter Ausnutzung der durch das Plasmid vermittelten Antibiotikaresistenzen bzw. -empfindlichkeiten selektiert und die Hybridvektoren amplifiziert. Nach Abtöten der Zellen werden die Hybridplasmide isoliert, mit den ursprünglich eingesetzten Restriktionsenzymen aufgeschnitten und die Genfragmente IFN-I, IFN-II und IFN-III durch Gelelektrophorese isoliert.
b) Verknüpfung der Genfragmente
Die durch Amplifikation erhaltenen Subfragmente IFN-I, IFN-II und IFN-III werden wie im Beispiel 2 beschrieben enzymatisch verknüpft und das so erhaltene synthetische Gen mit der DNA-Sequenz I in den Klonierungsvektor pUC 8 eingeführt. Man erhält ein Hybridplasmid gemäß Figur 4.

5. Synthese von Hybridplasmiden, die die DNA-Sequenz IA, IB und IC enthalten.

a) Hybridplasmid mit der Insertion IB
Das Hybridplasmid gemäß Figur 4, das die DNA-Sequenz I enthält, wird nach bekannten Methoden mit den Restriktionsenzymen Eco RI und Sal I geschnitten, das kleine Eco RI- und Sal I-Fragment durch Polyacrylamidgel-Elektrophorese abgetrennt und mit dem Enzym Ava II nachgeschnitten. Mit Hilfe des folgenden Adaptors

$$5' \text{ AA TTC ACC ATG} \qquad\qquad 3'$$
$$3' \qquad\quad \text{G TGG TAC CTG} \quad\ 5'$$
$$\text{Eco RI} \qquad\qquad\qquad \text{Ava II}$$

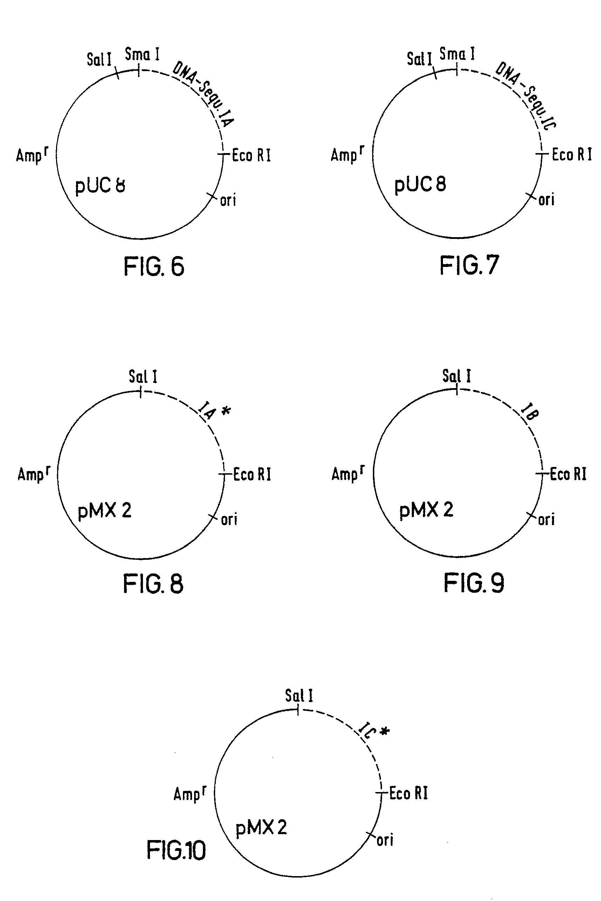

erhält man nach Ligation mit dem vorher erzeugten großen DNA-Fragment ein Hybridplasmid, das die DNA-Sequenz IB inseriert enthält (Figur 5).
b) Hybridplasmid mit der Insertion IA
Die DNA-Sequenz I wird mit dem Restriktionsenzym Pst I nach den Angaben des Herstellers geschnitten und das Eco RI-Pst I-Fragment isoliert. Daneben wird das käufliche Plasmid pUC 8 mit den Restriktionsenzymen Eco RI und Sma I geöffnet, und das vorher isolierte Fragment mit Hilfe des folgenden Adaptors

$$5' \qquad\qquad \text{GCT TAG TAA CCC} \quad 3'$$
$$3' \text{ A CGT CGA ATC ATT GGG} \quad 5'$$
$$\text{Pst I} \qquad\qquad\qquad\qquad \text{Sma I}$$

inseriert, wobei man ein Hybridplasmid gemäß Figur 6 erhält.
c) Hybridplasmid mit der Insertion IC
Das aus Beispiel 5a resultierende Hybridplasmid wird der Verdauung mit Eco RI und Pst I unterworfen. Das isolierte (Eco RI - Pst I)-Fragment wird analog 5b) zu einem neuen Hybridplasmid ligiert, welches nun die DNA-Sequenz IC inseriert enthält (Figur 7).

6. Konstruktion von Hybridplasmiden für die Expression der DNA-Sequenzen IA, IB und IC

a) Einbau in pKK 177.3
Das Expressionsplasmid pKK 177.3 (Plasmid ptac 11, Amman et al., Gene 25 (1983) 167, bei dem in die Eco RI-Erkennungsstelle synthetisch eine Sequenz eingebaut wurde, die eine Sal I-Schnittstelle enthält) wird mit den Restriktionsenzymen Eco RI und Sal I geöffnet. Aus dem Plasmid nach Fig. 5 wird die Insertion IB mit den Restriktionsenzymen Eco RI und Sal I herausgeschnitten. In gleicher Weise werden auch die (nur wenig verlängerten) Insertionen IA* und IC* isoliert, da nur wenige Nucleotide hinter dem eigentlichen Ende der beiden Genfragmente, gekennzeichnet durch die Schnittstelle des Restriktionsenzyms Sma I, im Plasmid pUC 8 eine Sal I-Schnittstelle sitzt (Figuren 6 und 7).

Die Fragmente IA*, IB und IC* werden auf 2%ige niedrig-schmelzende (low melting) Agarose gegeben, von der Plasmid-DNA abgetrennt und die Insertionen durch Auflösen des Gels bei erhöhter Temperatur (nach Maßgabe der Hersteller) wiedergewonnen. Durch Ligation des aufgeschnittenen Plasmids pKK 177.3 mit den Fragmenten IA* bzw. IB oder IC* wird ein Hybridplasmid geschaffen, bei dem jeweils den Insertionen eine Expressions- bzw. Regulationsregion vorgeschaltet ist. Nach Zugabe eines geeigneten Induktors wie Isopropyl-$\beta$-thiogalactopyranosid (IPTG) wird eine mRNA gebildet, die zur Expression der Methionyl-Polypeptide entsprechend den DNA-Sequenzen IA bzw. IB oder IC führt.

b) Einbau in pMX 2

Das Expressionsplasmid pMX 2 besteht aus einem um 21 Nucleotide verkürzten pUC 8-Plasmid und wird auf folgende Weise hergestellt:

pUC 8 wird mit der Restriktionsendonuclease Eco RI geöffnet und anschließend mit der Exonuclease Bal 31 unter Bedingungen behandelt, die die Abspaltung von etwa 20 Nucleotiden auf beiden Seiten der Eco RI-Schnittstelle erlauben (Maniatis a.a.O.). Anschließend werden eventuell überstehende Enden des so behandelten Plasmids mit Klenow-DNA-Polymerase aufgefüllt, das Plasmid mit der Restriktionsendonuclease Hind III nachgeschnitten und das Plasmid über 1%ige niedrig-schmelzende Agarosegele nach Maßgabe der Hersteller gereinigt. In das Plasmid wird der ursprünglich in pUC 8 vorhandene durch die Restriktionsenzymschnitte Eco RI und Hind III begrenzte Polylinker, der durch die zuvor beschriebenen Manipulationen zerstört wurde, reinseriert. Dazu wird pUC 8 mit dem Restriktionsenzym Eco RI geöffnet und die überstehenden Enden mit Klenow-DNA-Polymerase unter Verwendung von $^{32}$P-markierten Nucleosidtriphosphaten aufgefüllt. Der Polylinker wird anschließend aus dem Plasmid mit dem Restriktionsenzym Hind III herausgeschnitten und durch Elektrophorese auf 10% Acrylamidgelen vom Plasmid abgetrennt. Nach Identifikation der Polylinker-Bande mit Hilfe einer Autoradiographie wird der Polylinker durch Elektroelution von Acrylamidresten befreit und in das verkürzte pUC 8-Plasmid ligiert. Das so konstruierte Plasmid pMX 2 wird anschließend mit den Restriktionsenzymen Eco RI und Sal I geöffnet und mit den $\gamma$-Interferon-Genfragmenten IA* bzw. IB oder IC*, die an ihren Enden Eco RI- und Sal I-Erkennungssequenzen aufweisen, in das Expressionsplasmid pMX 2 ligiert (Fig. 8 bis 10). Klone, die einen hohen Interferontiter zeigen, werden dann anhand der biologischen Aktivitätsbestimmung identifiziert.

7. Transformation der Hybridplasmide.

Kompetente E. coli-Zellen werden mit 0,1 bis 1 µg der Hybridplasmide, die die Sequenzen IA oder IB oder IC enthalten, transformiert und auf Ampicillin enthaltenden Agarplatten plattiert. Anschließend lassen sich Klone, die korrekt integrierte $\gamma$-Interferongen-Sequenzen in den entsprechenden Plasmiden enthalten, durch DNA-Schnellaufarbeitung identifizieren (Maniatis a.a.O.).

8. Expression der $\gamma$-Interferon-Aktivität aufweisenden Polypeptide

Nach Transformation der genannten Hybridplasmide in E. coli werden Polypeptide exprimiert, die außer den entsprechenden $\gamma$-Interferon-Aminosäuresequenzen am Aminoterminus noch eine zusätzliche Methionylgruppe tragen, nämlich

bei der Konstruktion IA Met-(IFN-$\gamma$, Aminosäuren 1-127),

bei der Konstruktion IB Met-(IFN-$\gamma$, Aminosäuren 5-146),

und bei der Konstruktion IC Met-(IFN-$\gamma$, Aminosäuren 5-127).

9. Aufarbeitung und Reinigung

Die zur gewünschten optischen Dichte kultivierten Bakterienstämme werden mit einem geeigneten Induktor, beispielsweise IPTG, hinreichend lange, beispielsweise 2 Stunden, inkubiert. Anschließend werden die Zellen mit 0,1 % Kresol und 0,1 mM Benzylsulfonylfluorid abgetötet. Nach Zentrifugieren oder Filtrieren wird die Zellmasse in einer Pufferlösung (50 mM Tris, 50 mM EDTA, pH 7,5) aufgenommen und mechanisch aufgeschlossen, beispielsweise mit einer French-Presse bzw. $^{(R)}$DYNO-Mühle (Fa. Willy Bachofer, Basel), worauf die unlöslichen Bestandteile abzentrifugiert werden. Aus dem Überstand werden die $\gamma$-Interferon-Aktivität enthaltenden Proteine nach üblichen Verfahren gereinigt. Geeignet sind Ionenaustauscher-, Adsorptions-, Gelfiltrationssäulen oder Affinitätschromatographie an Antikörpersäulen.

Durch Natriumdodecylsulfat-Acrylamidgel- oder HPLC-Analytik werden Anreicherung und Reinheit der Produkte kontrolliert.

Zur biologischen Charakterisierung der Produkte auf γ-Interferonaktivität werden in bekanner Weise Indikatorzellinien, wie zum Beispiel Vero-Zellen, benutzt und mit Verdünnungsreihen interferonhaltiger Bakterienextrakte inkubiert. Anschließend wird durch Infektion mit einem Virus wie VSV (Vesicular Stomatitis Virus) überprüft, bis zu welchem Verdünnungsschritt durch Vorbehandlung mit dem Bakterienextrakt bei den Vero-Zellen ein antiviraler Status erzielt werden konnte. Die Auswertung kann mikroskopisch oder durch Bestimmung der Neutralrot-Aufnahme erfolgen.

Die Bestimmung der γ-Interferon-Aktivität kann auch mit einem handelsüblichen Radioimmunoassay (Celltech Ltd.), der auf einem monoklonalen Antikörper gegen γ-Interferon aufgebaut ist, erfolgen.

10. Modifikation der DNA-Sequenz

Zur Herstellung eines γ-Interferon-Analogen, bei dem in Position 102 anstelle von Serin Glutaminsäure steht, wird gemäß Beispiel 1 und 2 das folgende Nucleotid synthetisiert:

```
                         Glu
     5' AG CTT ACT AAC TAC GAA GTT ACT GAT TT
     3'    A TGA TTG ATG CTT CAA TGA CTA AA
       Hind III                      Aha III
```

Das Genfragment IFN-III wird mit dem Restriktionsenzym Aha III verdaut, das größere Fragment abgetrennt und mit dem obengenannten Nucleotid ligiert. Der Einbau in pUC 8 erfolgt gemäß Beispiel 3 c). Nach Transformation und Amplifikation gemäß Beispiel 4 a) wird die modifizierte Sequenz IFN-III durch Maxam-Gilbert-Sequenzierung verifiziert. Die Ligierung dieses modifizierten Subfragments mit den Genfragmenten IFN-I und IFN-II gemäß Beispiel 4 und das weitere Vorgehen nach Beispiel 5 bis 9 ergibt ein modifiziertes γ-IFN, bei dem in der Position 102 Glutaminsäure anstelle des Serins eingebaut ist. Dieses Produkt zeigt antivirale Aktivität.

DNA-Sequenz I *

| Triplett Nr. | | | | 0 | 1 | 2 | 3 | 4 | 5 |
|---|---|---|---|---|---|---|---|---|---|
| Aminosäure | | | | Met | Cys | Tyr | Cys | Gln | Asp |
| Nucleotid Nr. | | 1 | | | 10 | | | 20 | |
| Cod. Strang | 5' AA | TTC | ATG | TGC | TAC | TGC | CAG | GAC | |
| nicht cod. Strang | 3' | | G | TAC | ACG | ATG | ACG | GTC | CTG |

| 6 | 7 | 8 | 9 | 10 | 11 | 12 | 13 | 14 | 15 |
|---|---|---|---|---|---|---|---|---|---|
| Pro | Tyr | Val | Lys | Glu | Ala | Glu | Asn | Leu | Lys |
| | | 30 | 35 | | 40 | | | 50 | |
| CCG | TAC | GTT | AAA | GAA | GCT | GAA | AAC | CTG | AAA |
| GGC | ATG | CAA | TTT | CTT | CGA | CTT | TTG | GAC | TTT |

| 16 | 17 | 18 | 19 | 20 | 21 | 22 | 23 | 24 | 25 |
|---|---|---|---|---|---|---|---|---|---|
| Lys | Tyr | Phe | Asn | Ala | Gly | His | Ser | Asp | Val |
| | | 60 | | | 70 | | | 80 | |
| AAA | TAC | TTC | AAC | GCT | GGT | CAT | TCT | GAC | GTT |
| TTT | ATG | AAG | TTG | CGA | CCA | GTA | AGA | CTG | CAA |

| 26 | 27 | 28 | 29 | 30 | 31 | 32 | 33 | 34 | 35 |
|---|---|---|---|---|---|---|---|---|---|
| Ala | Asp | Asn | Gly | Thr | Leu | Phe | Leu | Gly | Ile |
| | | 90 | | | 100 | | | 110 | |
| GCT | GAC | AAT | GGT | ACT | CTG | TTC | CTG | GGG | ATC |
| CGA | CTG | TTA | CCA | TGA | GAC | AAG | GAC | CCC | TAG |

| 36 | 37 | 38 | 39 | 40 | 41 | 42 | 43 | 44 | 45 |
|---|---|---|---|---|---|---|---|---|---|
| Leu | Lys | Asn | Trp | Lys | Glu | Glu | Ser | Asp | Arg |
| | | 120 | | | 130 | | | 140 | |
| CTG | AAA | AAC | TGG | AAA | GAA | GAA | TCT | GAC | CGT |
| GAC | TTT | TTG | ACC | TTT | CTT | CTT | AGA | CTG | GCA |

* hier gezeigt mit der Sequenz entsprechend Eco RI und dem "Triplett-Nr. 0" am Aminoterminus und zwei Stop-Tripletts und der Sequenz für Sal I am Carboxyterminus

| 46 | 47 | 48 | 49 | 50 | 51 | 52 | 53 | 54 | 55 |
|---|---|---|---|---|---|---|---|---|---|
| Lys | Ile | Met | Gln | Ser | Gln | Ile | Val | Ser | Phe |
| | | 150 | | | 160 | | | 170 | |
| AAA | ATC | ATG | CAA | TCT | CAG | ATC | GTT | TCT | TTC |
| TTT | TAG | TAC | GTT | AGA | GTC | TAG | CAA | AGA | AAG |

| 56 | 57 | 58 | 59 | 60 | 61 | 62 | 63 | 64 | 65 |
|---|---|---|---|---|---|---|---|---|---|
| Tyr | Phe | Lys | Leu | Phe | Lys | Asn | Phe | Lys | Asp |
| | | 180 | | | 190 | | | 200 | |
| TAC | TTC | AAA | CTG | TTC | AAA | AAC | TTT | AAA | GAC |
| ATG | AAG | TTT | GAC | AAG | TTT | TTG | AAA | TTT | CTG |

| 66 | 67 | 68 | 69 | 70 | 71 | 72 | 73 | 74 | 75 |
|---|---|---|---|---|---|---|---|---|---|
| Asp | Gln | Ser | Ile | Gln | Lys | Ser | Val | Glu | Thr |
| | | 210 | | | 220 | | | 230 | |
| GAC | CAG | TCT | ATC | CAG | AAA | TCT | GTT | GAA | ACT |
| CTG | GTC | AGA | TAG | GTC | TTT | AGA | CAA | CTT | TGA |

| 76 | 77 | 78 | 79 | 80 | 81 | 82 | 83 | 84 | 85 |
|---|---|---|---|---|---|---|---|---|---|
| Ile | Lys | Glu | Asp | Met | Asn | Val | Lys | Phe | Phe |
| | | 240 | | | 250 | | | 260 | |
| ATC | AAG | GAA | GAC | ATG | AAC | GTT | AAA | TTT | TTC |
| TAG | TTC | CTT | CTG | TAC | TTG | CAA | TTT | AAA | AAG |

| 86 | 87 | 88 | 89 | 90 | 91 | 92 | 93 | 94 | 95 |
|---|---|---|---|---|---|---|---|---|---|
| Asn | Ser | Asn | Lys | Lys | Lys | Arg | Asp | Asp | Phe |
| | | 270 | | | 280 | | | 290 | |
| AAC | TCT | AAC | AAA | AAA | AAA | CGT | GAC | GAC | TTC |
| TTG | AGA | TTG | TTT | TTT | TTT | GCA | CTG | CTG | AAG |

| 96 | 97 | 98 | 99 | 100 | 101 | 102 | 103 | 104 | 105 |
|---|---|---|---|---|---|---|---|---|---|
| Glu | Lys | Leu | Thr | Asn | Tyr | Ser | Val | Thr | Asp |
| | | 300 | | | 310 | | | 320 | |
| GAA | AAG | CTT | ACT | AAC | TAC | TCT | GTT | ACT | GAT |
| CTT | TTC | GAA | TGA | TTG | ATG | AGA | CAA | TGA | CTA |

| 106 | 107 | 108 | 109 | 110 | 111 | 112 | 113 | 114 | 115 |
|-----|-----|-----|-----|-----|-----|-----|-----|-----|-----|
| Leu | Asn | Val | Gln | Arg | Lys | Ala | Ile | His | Glu |
|     |     | 330 |     |     | 340 |     |     | 350 |     |
| TTA | AAC | GTT | CAA | CGT | AAA | GCT | ATC | CAC | GAG |
| AAT | TTG | CAA | GTT | GCA | TTT | CGA | TAG | GTG | CTC |

| 116 | 117 | 118 | 119 | 120 | 121 | 122 | 123 | 124 | 125 |
|-----|-----|-----|-----|-----|-----|-----|-----|-----|-----|
| Leu | Ile | Gln | Val | Met | Ala | Glu | Leu | Ser | Pro |
|     |     | 360 |     |     | 370 |     |     | 380 |     |
| CTC | ATC | CAG | GTT | ATG | GCT | GAA | CTG | TCT | CCT |
| GAG | TAG | GTC | CAA | TAC | CGA | CTT | GAC | AGA | GGA |

| 126 | 127 | 128 | 129 | 130 | 131 | 132 | 133 | 134 | 135 |
|-----|-----|-----|-----|-----|-----|-----|-----|-----|-----|
| Ala | Ala | Lys | Thr | Gly | Lys | Arg | Lys | Arg | Ser |
|     |     | 390 |     |     | 400 |     |     | 410 |     |
| GCA | GCT | AAA | ACT | GGT | AAA | CGT | AAA | CGT | TCC |
| CGT | CGA | TTT | TGA | CCA | TTT | GCA | TTT | GCA | AGG |

| 136 | 137 | 138 | 139 | 140 | 141 | 142 | 143 | 144 | 145 |
|-----|-----|-----|-----|-----|-----|-----|-----|-----|-----|
| Gln | Met | Leu | Phe | Arg | Gly | Arg | Arg | Ala | Ser |
|     |     | 420 |     |     | 430 |     |     | 440 |     |
| CAG | ATG | CTG | TTC | CGC | GGT | CGT | CGT | GCT | TCT |
| GTC | TAC | GAC | AAG | GCG | CCA | GCA | GCA | CGA | AGA |

| 146 |     |     |     |     |     |
|-----|-----|-----|-----|-----|-----|
| Gln |     |     |     |     |     |
|     | 450 |     |     |     |     |
| CAG | TAA | TAG |     |     | 3' |
| GTC | ATT | ATC | AGC | T   | 5' |

DNA-Sequenz I A:*

|  |  |  | 0 |  |  |  |  |  |
|---|---|---|---|---|---|---|---|---|
|  |  |  | Met | (Aminosäuren 1–127) | Stp | Stp |  |  |
|  | 1 | 5 |  |  |  |  |  |  |
| 5' | AA | TTC | ATG | (Nucleotide 9–389) | TAG | TAA | CCC | 3' |
|  |  | G | TAC | (complementäre Nucleot.) | ATC | ATT | GGG |  |

DNA-Sequenz I B:*

|  |  |  | 0 |  |  |  |  |  |
|---|---|---|---|---|---|---|---|---|
|  |  |  | Met | (Aminosäuren 5–146) | Stp | Stp |  |  |
| 5' | AA | TTC | ATG | (Nucleotide 21–446) | TAA | TAG |  | 3' |
|  |  | G | TAC |  | ATT | ATC | AGC | T 5' |

DNA-Sequenz I C:*

|  |  |  | 0 |  |  |  |  |  |
|---|---|---|---|---|---|---|---|---|
|  |  |  | Met | (Aminosäuren 5–127) | Stp | Stp |  |  |
| 5' | AA | TTC | ATG | (Nucleotide 21–389) | TAG | TAA | CCC | 3' |
|  |  | G | TAC |  | ATC | ATT | GGG |  |

\* hier dargestellt mit der Sequenz für Eco RI und dem
"Triplett-Nr. 0" am Aminoterminus und zwei Stop-Codons und
den Sequenzen für Sma I bzw. Sal I am Carboxyterminus

12

DNA-Sequenz II

IFN-I:

```
                                               ┌─────────────── Ia ──────────────────┐
                          Met   Cys   Tyr   Cys   Gln   Asp
         5'   AA   TTC   ATG   TGC   TAC   TGC   CAG   GAC │
         3'          G   TAC   ACG   ATG   ACG   GTC   CTG
              Eco RI  ◄───────────────────── Ib ──────────
       ┌──────────────── Ic ──────────────►│◄─────────────
       Pro   Tyr   Val  ·Lys   Glu   Ala   Glu   Asn │ Leu   Lys
       CCG   TAC   GTT   AAA   GAA   GCT   GAA   AAC │ CTG   AAA
       GGC   ATG   CAA   TTT   CTT   CGA   CTT   TTG   GAC   TTT │
       ─────────────────►│◄──────────────────── Id ───────────
       ──────── Ie ─────────────────►│◄───────────── Ig ───────
       Lys   Tyr   Phe   Asn   Ala │ Gly   His   Ser   Asp   Val
       AAA   TAC   TTC   AAC   GCT │ GGT   CAT   TCT   GAC   GTT
       TTT   ATG   AAG   TTG   CGA   CCA   GTA │ AGA   CTG   CAA
       ◄──── If ─────────────────────►│◄──── Ih ───────────
       ─────────────────►│◄──────────────── Ii ──────────►
       Ala   Asp │ Asn   Gly   Thr   Leu   Phe   Leu  (Gly)
       GCT   GAC │ AAT   GGT   ACT   CTG   TTC   CTG   GG
       CGA   CTG   TTA   CCA │ TGA   GAC   AAG   GAC   CCC   TAG
       ─────────────────►│◄──────── Ij ──────────►
                                              Bam HI
```

DNA-Sequenz II

IFN-II:

```
                              ←───────────────── IIa ─────────────────────→  │
                                                                             │←─

(Gly) Ile Leu   Lys    Asn    Trp   Lys   Glu    Glu    Ser    Asp  │ Arg

    G ATC CTG   AAA    AAC    TGG   AAA   GAA    GAA    TCT    GAC  │ CGT

  BamHI   GAC   TTT    TTG    ACC   TTT   CTT    CTT    AGA    CTG  │ GCA
              ←───────────────── IIb ──────────────────
         ─────────────────── IIc ──────────────────────────────→│
 Lys    Ile    Met    Gln    Ser   Gln    Ile    Val    Ser    Phe │

 AAA    ATC    ATG    CAA    TCT   CAG    ATC    GTT    TCT    TTC │

 TTT  │ TAG    TAC    GTT    AGA   GTC    TAG    CAA    AGA    AAG
      │←─────────────────── IId ─────────────────────────
    ←─────────────────── IIe ──────────────────────────────→│

 Tyr    Phe    Lys    Leu    Phe   Lys    Asn    Phe    Lys    Asp │

 TAC    TTC    AAA    CTG    TTC   AAA    AAC    TTT    AAA    GAC │

 ATG    AAG    TTT    GAC    AAG   TTT    TTG    AAA    TTT    CTG
 ───────────────→│←─────────────────── IIf ──────────────────
    ←─────────────────── IIg ──────────────────────────────

 Asp    Gln    Ser    Ile    Gln   Lys    Ser    Val    Glu    Thr

 GAC    CAG    TCT    ATC    CAG   AAA    TCT    GTT    GAA    ACT

 CTG    GTC    AGA    TAG    GTC   TTT    AGA    CAA    CTT    TGA
 ───────────────────→│←─────────────── IIh ──────────────────
 ──────────────→│←──────────── IIi ──────────────
 Ile  │ Lys    Glu    Asp    Met   Asn    Val    Lys    Phe    Phe

 ATC  │ AAG    GAA    GAC    ATG   AAC    GTT    AAA    TTT    TTC

 TAG    TTC    CTT    CTG    TAC   TTG    CAA    TTT    AAA    AAG
 ───────────────────→│←──────────────── IIj ──────────────────
 ──────────────→│←──────────────── IIk ──────────────────

 Asn  │ Ser    Asn    Lys    Lys   Lys    Arg    Asp    Asp    Phe

 AAC  │ TCT    AAC    AAA    AAA   AAA    CGT    GAC    GAC    TTC

 TTG    AGA    TTG    TTT    TTT   TTT    GCA    CTG    CTG    AAG
 ───────────────────→│←────────────────── IIl ──────────────────
 ─────────────→│
 Glu    (Lys)

 GAA      A

 CTT    TTC    GA    Hind III
 ──────────────────→
```

DNA-Sequenz II

IFN-III:

```
                                              ┌──────────── IIIa ──────────────→┐←─
              (Lys) Leu     Thr    Asn    Tyr    Ser    Val    Thr │ Asp
                AG  CTT     ACT    AAC    TAC    TCT    GTT    ACT │ GAT
       Hind III       A     TGA    TTG    ATG    AGA    CAA    TGA │ CTA
                   ←──────────────────── IIIb ─────────────────┘
        ┌─────────────────────── IIIc ──────────────────────→┐┌──
       Leu    Asn    Val    Gln    Arg    Lys    Ala    Ile │ His    Glu
       TTA    AAC    GTT    CAA    CGT    AAA    GCT    ATC │ CAC    GAG
     ┌ AAT    TTG    CAA    GTT    GCA    TTT    CGA    TAG │ GTG    CTC
     └─────────────────────── IIId ──────────────────→┘←─
       ┌─────────────────────── IIIe ──────────────────→┐┌──
       Leu    Ile    Gln    Val    Met    Ala    Glu │ Leu    Ser    Pro
       CTC    ATC    CAG    GTT    ATG    GCT    GAA │ CTG    TCT    CCT
     │ GAG    TAG    GTC    CAA    TAC    CGA    CTT │ GAC    AGA    GGA
     └←──────────────────── IIIf ────────────────────→┘←─
        ┌────────── IIIg ──────────→┐┌──────────── IIIi ──────────
       Ala    Ala    Lys    Thr    Gly    Lys │ Arg    Lys    Arg    Ser
       GCA    GCT    AAA    ACT    GGT    AAA │ CGT    AAA    CGT    TCC
       CGT    CGA    TTT    TGA    CCA    TTT │ GCA    TTT │ GCA    AGG
        └────────────── IIIh ─────────────→┘←─          │
        ┌──────── IIIi ────────→┐┌──────────── IIIk ──────────
       Gln    Met    Leu    Phe    Arg │ Gly    Arg │ Arg    Ala    Ser
       CAG    ATG    CTG    TTC    CGC │ GGT    CGT │ CGT    GCT    TCT
       GTC    TAC    GAC    AAG    GCG │ CCA    GCA │ GCA    CGA    AGA
        └──────── IIIj ────────→┘←─          └──────── IIIl ──────
        ┌──────────→
       Gln    Stp    Stp
       CAG    TAA    TAG                        3'
       GTC    ATT    ATC    AGC    T            5'
        └──────────────────────────→

                      Sal I
```

**Patentansprüche**
**Patentansprüche für folgende Vertragsstaaten: BE, CH, DE, FR, GB, IT, LI, LU, NL, SE**

1. Polypeptid mit der Teilsequenz des Human-Gamminterferons (IFN-$\gamma$), bestehend aus der Aminosäuresequenz 5 bis 146.

2. Analoga des Human-Gammainterferons gemäß Anspruch 1 mit der antiviralen, antitumoralen und

EP 0 161 504 B1

antineoplastischen Aktivität von IFN-$\gamma$, bei denen die natürlichen Aminosäuresequenz durch Austausch, Einfügung oder Eliminierung von Aminosäuren abgewandelt wurde, unter Erhaltung des N-Terminus Asp$^5$.

3. Gentechnologisches Verfahren zur Herstellung der Polypeptide nach Anspruch 1 oder 2, dadurch gekennzeichnet, daß man in einer Wirtszelle, vorzugsweise E. coli, ein Gen mit einer DNA der allgemeinen Formel

Pr - R - Z

in der Pr und R für Nucleotidsequenzen stehen, die für die folgenden Aminosäuresequenzen bzw. Aminosäure codieren:

Pr    für ein Präpeptid, das im Wirtsorganismus oder in vitro abgespalten wird, oder Met,
R    für die Aminosäuren 5 bis 146 oder Modifikationen dieser Aminosäuresequenz und
Z    ein oder vorzugsweise zwei Stop-Codon(s) bedeutet, zur Expression bringt.

4. DNA-Sequenz der Formel

(IFN-$\gamma$ 5-146)    (IB)

in denen (IFN-$\gamma$ 5-146) die Codons für die Aminosäuren 5-146 von IFN-$\gamma$ oder Modifikationen dieser Aminosäuresequenz bedeutet.

5. Hybridplasmide, die die DNA-Sequenz IB gemäß Anspruch 4 enthalten.

6. Hybridplasmide, die zwischen einer Eco RI- und einer Sal I-Schnittstelle die DNA-Sequenz (IB) gemäß Anspruch 4 enthalten.

7. Hybridplasmide nach Anspruch 6, die zwischen der Eco RI-Schnittstelle und dem Codon für die ersten Aminosäuren der Interferon-Sequenz eine Präsequenz enthalten.

8. Wirtszellen, vorzugsweise E. coli, die Hybridplasmide nach Anspruch 5 bis 7 enthalten.

9. Arzneimittel, gekennzeichnet durch einen Gehalt an einem Polypeptid nach Anspruch 1 oder 2.

**Patentansprüche für folgenden Vertragsstaat: AT**

1. Gentechnologisches Verfahren zur Herstellung von Polypeptiden mit der Teilsequenz des Human-Gammainterferons (IFN-$\gamma$), bestehend aus den Aminosäuresequenzen 5 bis 146 bzw. von Analoga des Human-Gammainterferons mit diesen Teilsequenzen und mit der antiviralen, antitumoralen und antineoplastischen Aktivität von IFN-$\gamma$, bei denen die natürliche Aminosäuresequenz durch Austausch, Einfügung und Eliminierung von Aminosäuren abgewandelt wurde unter Erhaltung des N-Terminus Asp$^5$, dadurch gekennzeichnet, daß man in einem Wirtsorganismus ein Gen mit einer DNA der allgemeinen Formel

Pr - R - Z

in der Pr und R für Nucleotidsequenzen stehen, die für die folgenden Aminosäuren bzw. Aminosäuresequenzen codieren:

Pr    steht für ein Praepeptid, das im Wirtsorganismus oder in vitro abgespalten wird, oder Met,
R    für die Aminosäuren 5 bis 146 oder Modifikationen dieser Aminosäuresequenz, und
Z    ein oder vorzugsweise zwei Stop-Codon(s) bedeutet,
zur Expression bringt.

2. Verfahren nach Anspruch 1, dadurch gekennzeichnet, daß Z für zwei Stop-Codons steht.

3. Verfahren nach Anspruch 1 oder 2, dadurch gekennzeichnet, daß der Wirtsorganismus E. coli ist.

16

EP 0 161 504 B1

## Claims
## Claims for the following Contracting States: BE, CH, DE, FR, GB, IT, LI, LU, NL, SE

1. A polypeptide having the partial sequence of human gamma interferon (IFN-$\gamma$), comprising amino-acid sequence 5 to 146.

2. Analogs of human gamma interferon as claimed in Claim 1, having the antiviral, antitumor and antineoplastic activity of IFN-$\gamma$, in which the natural amino-acid sequence has been modified by exchange, introduction or elimination of amino acids, while retaining the N-terminal Asp[5].

3. A genetic engineering process for the preparation of the polypeptides as claimed in Claim 1 or 2, which comprises bringing about the expression in a host organism, preferably E. coli, of a gene having a DNA of the formula

   Pr - R - Z

   in which Pr and R represent nucleotide sequences which code for the following amino-acid sequences or amino acid:
   Pr for a prepeptide which is eliminated in the host organism or in vitro, or Met,
   R for amino acids 5 to 146 or modifications of this amino-acid sequence, and
   Z denotes one or preferably two stop codon(s).

4. The DNA sequence of the formula

   (IFN-$\gamma$ 5-146)      (IB)

   in which (IFN-$\gamma$ 5-146) denotes the codons for amino acids 5-146 of IFN-$\gamma$ or modifications of this amino-acid sequence.

5. A hybrid plasmid which contains the DNA sequence IB as claimed in Claim 4.

6. A hybrid plasmid which contains between an Eco RI cleavage site and a Sal I cleavage site, the DNA sequence (IB) as claimed in Claim 4.

7. A hybrid plasmid as claimed in Claim 6, which contains a presequence between the Eco RI cleavage site and the codon for the first amino acids of the interferon sequence.

8. A host cell, preferably E. coli, which contains hybrid plasmids as claimed in Claims 5 to 7.

9. A medicament containing a polypeptide as claimed in Claim 1 or 2.

## Claims for the following Contracting State: AT

1. A genetic engineering process for the preparation of polypeptides having the partial sequence of human gamma interferon (IFN-$\gamma$), composed of amino-acid sequences 5 to 146, or of analogs of human gamma interferon having these partial sequences and the antiviral, antitumor and aminoneoplastic activity of IFN-$\gamma$, in which the natural amino-acid sequence has been modified by exchange, introduction or elimination of amino acids, while retaining the N-terminal Asp[5], which comprises bringing about the expression in a host organism of a gene having a DNA of the formula

   Pr - R - Z

   in which Pr and R represent nucleotide sequences which code for the following amino acids or amino-acid sequences:
   Pr for a prepeptide which is eliminated in the host organism or in vitro, or Met,
   R for amino acids 5 to 146 or modifications of this amino-acid sequence, and
   Z denotes one or preferably two stop codon(s).

17

2. The process as claimed in Claim 1, wherein Z represents two stop codons.

3. The process as claimed in Claim 1 or 2, wherein the host organism is E. coli.

**Revendications**
**Revendications pour les Etats contractants suivants: BE, CH, DE, FR, GB, IT, LI, LU, NL, SE**

1. Polypeptide comportant la séquence partielle de l'interféron gamma humain (IFN-$\gamma$), constitué par la séquence d'acides aminés 5 à 146.

2. Analogues de l'interféron gamma humain selon la revendication 1, présentant l'activité antivirale, antitumorale et antinéoplasique de IFN-$\gamma$, dont la séquence naturelle des acides aminés est modifiée par échange, incorporation ou élimination d'acides aminés, avec maintien de Asp$^5$ du côté N terminal.

3. Procédé de préparation par génie génétique des polypeptides selon l'une des revendications 1 ou 2, caractérisé en ce que l'on exprime dans une cellule hôte, de préférence E. coli, un gène avec un ADN de formule générale

Pr-R-Z

dans laquelle Pr et R représentent des séquences nucléotidiques, qui sont codent pour les séquences d'acides aminés ou les acides aminés suivants:
Pr, pour un prépeptide, qui est détaché par coupure in vitro ou dans l'organisme hôte, ou bien pour Met,
R, pour les acides aminés 5 à 146 ou des modifications de cette séquence d'acides aminés, et
Z représente 1 ou de préférence 2 codon(s) d'arrêt.

4. Séquence d'ADN de formule

(IFN-$\gamma$ 5-146)    (IB)

dans laquelle (IFN-$\gamma$ 5-146) représente les codons pour les acides aminés 5-146 de IFN-$\gamma$ ou des modifications de cette séquence d'acides aminés.

5. Plasmides hybrides, qui renferment la séquence d'ADN IB selon la revendication 4.

6. Plasmides hybrides, qui, entre une position de coupure par Eco RI et une position de coupure par Sal-I, renferment la séquence d'ADN IB selon la revendication 4.

7. Plasmides hybrides selon la revendication 6, qui, entre la position de coupure par Eco RI et le codon pour les premiers acides aminés de la séquence d'interféron, renferment une préséquence.

8. Cellules hôtes, de préférence E. coli, qui renferment les plasmides hybrides selon les revendications 5 à 7.

9. Médicaments, caractérisés en ce qu'ils renferment un polypeptide selon l'une des revendications 1 ou 2.

**Revendications pour l'Etat contractant suivant: AT**

1. Procédé de préparation par génie génétique des polypeptides comportant la séquence partielle de l'interféron gamma humain (IFN-$\gamma$), constitués par les séquences d'acides aminés 5 à 146, ou d'analogues de l'interféron gamma humain pourvus de ces séquences partielles et de l'activité antivirale, antitumorale et antinéoplasique de IFN-$\gamma$, dont la séquence naturelle des acides aminés est modifiée par échange, incorporation ou élimination d'acides aminés, avec maintien de Asp$^5$ sur le N terminal, caractérisé en ce que l'on exprime, dans une cellule hôte, un gène avec un ADN de formule générale

Pr-R-Z

dans laquelle Pr et R représentent des séquences nucléotidiques, qui codent pour les acides aminés ou les séquences d'acides aminés suivants:

Pr, pour un prépeptide, qui est détaché par coupure in vitro ou dans l'organisme hôte, ou bien pour Met,

R, pour les acides aminés 5 à 146 ou des modifications de cette séquence d'acides aminés, et

Z représente 1 ou de préférence 2 codon(s) d'arrêt.

2. Procédé selon la revendication 1, caractérisé en ce que Z représente deux codons d'arrêt.

3. Procédé selon l'une des revendications 1 ou 2, caractérisé en ce que l'organisme hôte est E. coli.

FIG.1

FIG.2

FIG.3

FIG.4

FIG.5

FIG. 6

FIG. 7

FIG. 8

FIG. 9

FIG.10